# EUROPEAN PATENT SPECIFICATION

(11) **EP 2 860 261 B1**
(45) Date of publication and mention of the grant of the patent: **12.09.2018**
(21) Application number: 12877468.4
(22) Date of filing: 23.05.2012
(51) Int. Cl.: C12Q 1/68, C12N 15/10

(54) **METHOD AND SYSTEM FOR IDENTIFYING TYPES OF TWINS**
VERFAHREN UND SYSTEM ZUR IDENTIFIZIERUNG VON ZWILLINGSTYPEN
PROCÉDÉ ET SYSTÈME POUR IDENTIFIER DES TYPES DE JUMEAUX

(43) Date of publication of application: 15.04.2015
(73) Proprietor: BGI Genomics Co., Ltd., Yantian District Shenzhen, Guangdong Province 518083 (CN)
(72) Inventor: GE, Huijuan, Shenzhen Guangdong 518083 (CN); ZHENG, Jing, Shenzhen Guangdong 518083 (CN); YI, Shang, Shenzhen Guangdong 518083 (CN); LI, Xuchao, Shenzhen Guangdong 518083 (CN); WANG, Jian, Shenzhen Guangdong 518083 (CN); WANG, Jun, Shenzhen Guangdong 518083 (CN); YANG, Huanming, Shenzhen Guangdong 518083 (CN); ZHANG, Xiuqing, Shenzhen Guangdong 518083 (CN)
(74) Representative: Kador & Partner PartG mbB
(86) International application number: PCT/CN2012/075969
(87) International publication number: WO 2013/173993

(56) References cited:
- WO-A1-03/031646
- WO-A1-2011/090556
- CN-A- 102 325 901
- GB-A- 2 484 764
- M.-J. YANG: "Determination of twin zygosity using a commercially available STR analysis of 15 unlinked loci and the gender-determining marker amelogenin - a preliminary report", HUMAN REPRODUCTION, vol. 21, no. 8, 9 May 2006 (2006-05-09), pages 2175-2179, XP55138565, ISSN: 0268-1161, DOI: 10.1093/humrep/del133
- ROMAIN GUILHERME ET AL: "Zygosity and chorionicity in triplet pregnancies: new data", HUMAN REPRODUCTION, OXFORD JOURNALS, GB , vol. 24, no. 1 1 January 2009 (2009-01-01), pages 100-105, XP003027786, ISSN: 0268-1161, DOI: 10.1093/HUMREP/DEN364 Retrieved from the Internet: URL:http://humrep.oxfordjournals.org/conte nt/24/1/100 [retrieved on 2008-10-22]
- GARY J W LIAO ET AL: "Targeted massively parallel sequencing of maternal plasma DNA permits efficient and unbiased detection of fetal alleles", CLINICAL CHEMISTRY, AMERICAN ASSOCIATION FOR CLINICAL CHEMISTRY, WASHINGTON, DC , vol. 57, no. 1 1 January 2011 (2011-01-01), pages 92-101, XP002674771, ISSN: 0009-9147, DOI: 10.1373/CLINCHEM.2010.154336 Retrieved from the Internet: URL:http://www.clinchem.org/content/57/1/9 2 [retrieved on 2010-11-15]
- WANG, LIN ET AL.: 'Use of PCR-STR technique in zygosity diagnosis of twins' CHINESE JOURNAL OF CURRENT CLINICAL MEDICINE vol. 7, no. 5, 2009, pages 385 - 388, XP008175694

### SEQUENCE LISTING

<110> BGI Diagnosis Co., Ltd. BGI SHENZHEN
<120> METHOD AND SYSTEM FOR IDENTIFYING TYPES OF TWINS
<130> PIDC120705PEP
<160> 34
<170> PatentIn version 3.3
<210> 1
   <211> 26
   <212> DNA
   <213> Artificial
<220>
   <223> primer
<400> 1
   aataatcagt atgtgacttg gattga 26
<210> 2
   <211> 22
   <212> DNA
   <213> Artificial
<220>
   <223> primer
<400> 2
   ataggatgga tggatagatg ga 22
<210> 3
   <211> 22
   <212> DNA
   <213> Artificial
<220>
   <223> primer
<400> 3
   cttagggaac cctcactgaa tg 22
<210> 4
   <211> 22
   <212> DNA
   <213> Artificial
<220>
   <223> primer
<400> 4
   gtccttgtca gcgtttattt gc 22
<210> 5
   <211> 21
   <212> DNA
   <213> Artificial
<220>
   <223> primer
<400> 5
   cagagcaaga ccctgtctca t 21
<210> 6
   <211> 21
   <212> DNA
   <213> Artificial
<220>
   <223> primer
<400> 6
   tcaacagagg cttgcatgta t 21
<210> 7
   <211> 21
   <212> DNA
   <213> Artificial
<220>
   <223> primer
<400> 7
   atacagacag acagacaggt g 21
<210> 8
   <211> 23
   <212> DNA
   <213> Artificial
<220>
   <223> primer
<400> 8
   gcatgtatct atcatccatc tct 23
<210> 9
   <211> 20
   <212> DNA
   <213> Artificial
<220>
   <223> primer
<400> 9
   gggtgatttt cctctttggt 20
<210> 10
   <211> 33
   <212> DNA
   <213> Artificial
<220>
   <223> primer
<400> 10
   aacatttgta tctttatctg tatccttatt tat 33
<210> 11
   <211> 23
   <212> DNA
   <213> Artificial
<220>
   <223> primer
<400> 11
   acagtaactg ccttcataga tag 23
<210> 12
   <211> 22
   <212> DNA
   <213> Artificial
<220>
   <223> primer
<400> 12
   gtgtcagacc ctgttctaag ta 22
<210> 13
   <211> 20
   <212> DNA
   <213> Artificial
<220>
   <223> primer
<400> 13
   acaatgaggt gcaagaatgt 20
<210> 14
   <211> 20
   <212> DNA
   <213> Artificial
<220>
   <223> primer
<400> 14
   gttagatggg tggatggata 20
<210> 15
   <211> 20
   <212> DNA
   <213> Artificial
<220>
   <223> primer
<400> 15
   tggaaacaga aatggcttgg 20
<210> 16
   <211> 20
   <212> DNA
   <213> Artificial
<220>
   <223> primer
<400> 16
   gattgcagga gggaaggaag 20
<210> 17
   <211> 27
   <212> DNA
   <213> Artificial
<220>
   <223> primer
<400> 17
   tttgtatttc atgtgtacat tcgtatc 27
<210> 18
   <211> 28
   <212> DNA
   <213> Artificial
<220>
   <223> primer
<400> 18
   acctatcctg tagattattt tcactgtg 28
<210> 19
   <211> 20
   <212> DNA
   <213> Artificial
<220>
   <223> primer
<400> 19
   tctgacccat ctaacgccta 20
<210> 20
   <211> 27
   <212> DNA
   <213> Artificial
<220>
   <223> primer
<400> 20
   cagacagaaa gatagataga tgattga 27
<210> 21
   <211> 21
   <212> DNA
   <213> Artificial
<220>
   <223> primer
<400> 21
   atgtacatgt gtctgggaag g 21
<210> 22
   <211> 18
   <212> DNA
   <213> Artificial
<220>
   <223> primer
<400> 22
   tactgccaac acttgtcc 18
<210> 23
   <211> 22
   <212> DNA
   <213> Artificial
<220>
   <223> primer
<400> 23
   tcgctcgctc tatctatcta tc 22
<210> 24
   <211> 19
   <212> DNA
   <213> Artificial
<220>
   <223> primer
<400> 24
   acagaaccaa taagatgag 19
<210> 25
   <211> 19
   <212> DNA
   <213> Artificial
<220>
   <223> primer
<400> 25
   agactcagtc tcaaagaaa 19
<210> 26
   <211> 18
   <212> DNA
   <213> Artificial
<220>
   <223> primer
<400> 26
   attgagaaaa ctccttac 18
<210> 27
   <211> 20
   <212> DNA
   <213> Artificial
<220>
   <223> primer
<400> 27
   actactccaa ggttccagcc 20
<210> 28
   <211> 18
   <212> DNA
   <213> Artificial
<220>
   <223> primer
<400> 28
   tgtggtagat agatgata 18
<210> 29
   <211> 23
   <212> DNA
   <213> Artificial
<220>
   <223> primer
<400> 29
   ggatgcatag gtagatagat aga 23
<210> 30
   <211> 20
   <212> DNA
   <213> Artificial
<220>
   <223> primer
<400> 30
   taataaatcc cctctcatct 20
<210> 31
   <211> 20
   <212> DNA
   <213> Artificial
<220>
   <223> primer
<400> 31
   caaggatggg tggatcaata 20
<210> 32
   <211> 20
   <212> DNA
   <213> Artificial
<220>
   <223> primer
<400> 32
   ttgtatgagc cacttcccat 20
<210> 33
   <211> 20
   <212> DNA
   <213> Artificial
<220>
   <223> primer
<400> 33
   gcctgggcaa cagaataaga 20
<210> 34
   <211> 19
   <212> DNA
   <213> Artificial
<220>
   <223> primer
<400> 34
   atcttctctc tttcttcct 19

## Description

### TECHNICAL FIELD

Aspects of the present disclosure generally relate to a field of bioinformatics, particularly to a method of determining a genotype of twins and a system thereof, more particularly to a method of determining whether twins are fraternal twins and a system thereof.

### BACKGROUND

In general, normal female ovulate one egg for one time, which develops into an embryo after normal fertilization. However, sometimes a single fertilized egg segregates into two parts which separately develop into two embryos due to some unknown reasons, i.e., twins. Such twins deriving from the single fertilized egg carry an identical genetic background, of which have an identical gender, an identical appearance, etc. If one fetus of such twins suffers a certain genetic disease, then the other fetus is also bound to suffer the same certain genetic disease. In general, based on different segregation time, a placenta of the identical twins may be a mono-chorionic and mono-amnionic type, a mono-chorionic and di-amnionic type, and a di-chorionic and di-amnionic type. In some cases, female ovulate more than one egg for one time. If two ovulated eggs both are normally-fertilized and further develop into two embryos, i.e., fraternal twins. Fetuses of the fraternal twins randomly inherit parental genomes in accordance with Mendelian, resulting in carrying genes not in identical types, by which the fraternal twins may have a different gender and a different appearance, and may also carry different genetic diseases. Twins in the whole world have an average birth rate of 1:89. Whether born twins are monozygotic may be preliminary determined based on their gender and appearance with an unreliable result, which still needs further determination in virtue of other genetic factors such as a blood type and etc. However, the fraternal twins may also be a di-chorionic and di-amnionic type or a mono-chorionic and di-amnionic type, thus, whether twins are the fraternal twins may not be determined only based on a B ultrasonic result showing the di-chorionic and di-amnionic type. Even though fetuses of twins may be respectively subjected to prenatal diagnosis based on an amniocentesis result, a sampling method thereof may cause a certain probability of abortion. At same time, the method of subjecting the two fetuses of the twins to amniocentesis has a great difficulty, which easily fails.

Currently, the method of determining fraternal twins in prenatal diagnosis still needs to be improved.

### SUMMARY

Embodiments of the present disclosure seek to solve at least one of the problems existing in the related art to at least some extent.

Therefore, the present disclosure provides a means of determining whether twins are fraternal twins

The scope of the invention is defined by the appended claims. Embodiments of a first broad aspect of the present disclosure provide a method of determining whether twins are fraternal twins. According to embodiments of the present disclosure, the method may comprise following steps: genotyping at least one polymorphic site of fetuses of the twins, to obtain polymorphism type of the fetuses; comparing the polymorphism type of the fetuses with corresponding polymorphic site of parents thereof; and determining whether the twins are the fraternal twins based on a comparing result. Using the method, whether the twins are the fraternal twins may be effectively determined. Taken STR as an example, a paternal genetic STR genotype is determined in a plasma in view of pregnant STR genotype, if the paternal genetic STR genotype in a certain site is heterozygous and totally different with that of the pregnant STR genotype, and both two of the paternal genotypes can be detected in the plasma, then the twins may be determined as fraternal twins.

According to embodiments of the present disclosure, the above method of determining whether twins are fraternal twins may also have following additional technique features:
In an embodiment of the present disclosure, the step of genotyping at least one polymorphic site of the fetuses of the twins may further comprise: extracting a nucleic acid sample of the fetuses from a pregnant sample; constructing a sequencing library for the nucleic acid sample of the fetuses; subjecting the sequencing library to sequencing, to obtain a sequencing result; and determining the polymorphism type of the fetuses based on the sequencing result. Then, it may effectively obtain the polymorphism type of the fetuses by sampling from a pregnant woman, by which whether the twins are the fraternal twins may be further effectively determined.

In an embodiment of the present disclosure, the pregnant sample is selected from a group consisting of peripheral blood, pregnant woman urine and breast milk. Thus, it may effectively obtain the polymorphism type of the fetuses by noninvasive sampling from a pregnant woman, by which whether the twins are the fraternal twins may be further effectively determined, without causing adverse effects to fetal growth.

In an embodiment of the present disclosure, prior to the step of constructing the sequencing library, the method may further comprise: subjecting the nucleic acid sample of the fetuses to amplifying, to obtain an amplification product containing the polymorphic site, for constructing the sequencing library. Preferably, the amplification product has a length of 150 bp or less. Thus, it may further improve efficiency of determining whether the twins are the fraternal twins.

In an embodiment of the present disclosure, the polymorphism is selected from a group consisting of STR, VNTR, RFLP, STS, SSCP, CAPS and SNP. In an embodiment of the present disclosure, the polymorphic site is an STR polymorphic site being at least one selected from a group consisting of vWA, TPOX, D3S1358, D16S539, D5S818, CSF1PO, D11S2368, D2S1338, D8S1179, D13S317, D21S1437, D16S3391, Penta E, D12S1064, D12S391, D6S1043 and D19S433. Thus, it may further improve efficiency of determining whether the twins are the fraternal twins.

In an embodiment of the present disclosure, the nucleic acid sample of the fetuses is subjected to amplifying by PCR, and for vWA, oligonucleotides shown as SEQ ID NO: 1 and SEQ ID NO: 2 are used as PCR primers; for TPOX, oligonucleotides shown as SEQ ID NO: 3 and SEQ ID NO: 4 are used as PCR primers; for D3S1358, oligonucleotides shown as SEQ ID NO: 5 and SEQ ID NO: 6 are used as PCR primers; for D16S539, oligonucleotides shown as SEQ ID NO: 7 and SEQ ID NO: 8 are used as PCR primers; for D5S818, oligonucleotides shown as SEQ ID NO: 9 and SEQ ID NO: 10 are used as PCR primers; for CSF1PO, oligonucleotides shown as SEQ ID NO: 11 and SEQ ID NO: 12 are used as PCR primers; for D11S2368, oligonucleotides shown as SEQ ID NO: 13 and SEQ ID NO: 14 are used as PCR primers; for D2S1338, oligonucleotides shown as SEQ ID NO: 15 and SEQ ID NO: 16 are used as PCR primers; for D8S1179, oligonucleotides shown as SEQ ID NO: 17 and SEQ ID NO: 18 are used as PCR primers; for D13S317, oligonucleotides shown as SEQ ID NO: 19 and SEQ ID NO: 20 are used as PCR primers; for D21S1437, oligonucleotides shown as SEQ ID NO: 21 and SEQ ID NO: 22 are used as PCR primers; for D16S3391, oligonucleotides shown as SEQ ID NO: 23 and SEQ ID NO: 24 are used as PCR primers; for Penta E, oligonucleotides shown as SEQ ID NO: 25 and SEQ ID NO: 26 are used as PCR primers; for D12S1064, oligonucleotides shown as SEQ ID NO: 27 and SEQ ID NO: 28 are used as PCR primers; for D12S391, oligonucleotides shown as SEQ ID NO: 29 and SEQ ID NO: 30 are used as PCR primers; for D6S1043, oligonucleotides shown as SEQ ID NO: 31 and SEQ ID NO: 32 are used as PCR primers; for D19S433, oligonucleotides shown as SEQ ID NO: 33 and SEQ ID NO: 34 are used as PCR primers. Thus, it may further improve efficiency of determining whether the twins are the fraternal twins.

Embodiments of a second broad aspect of the present disclosure provide a system for determining whether twins are fraternal twins. According to embodiments of the present disclosure, the system may comprise: a genotyping apparatus, for subjecting at least one polymorphic site of fetuses of the twins to genotyping, to obtain polymorphism type of the fetuses; an analyzing apparatus, connected with the genotyping apparatus, and suitable for comparing the polymorphism type of the fetuses with corresponding polymorphic site of parents thereof; and determining whether the twins are the fraternal twins based on a comparing result. Using the system may effectively implement the above-mentioned method of determining whether twins are fraternal twins. Thus, the system may effectively determine whether twins are fraternal twins. Taken STR as an example, a paternal genetic STR genotype is determined in a plasma in view of pregnant STR genotype, if the paternal genetic STR genotype in a certain site is heterozygous and totally different with that of the pregnant STR genotype, and both two of the paternal genotypes can be detected in the plasma, then the twins may be determined as fraternal twins.

According to embodiments of the present disclosure, the above system for determining whether twins are fraternal twins may further comprise following additional technical features:
According to an embodiment of the present disclosure, the genotyping apparatus may further comprise: a nucleic acid extracting unit, for extracting a nucleic acid sample of the fetuses from a pregnant sample; a library constructing unit, connected to the nucleic acid extracting unit, and suitable for constructing a sequencing library for the nucleic. acid sample of the fetuses; a sequencing unit, connected to the library constructing unit, and suitable for subjecting the sequencing library sequencing, to obtain a sequencing result; and a genotype determining unit, connected to the sequencing unit, and suitable for determining the polymorphism type of the fetuses. Thus, it may further improve efficiency of determining whether twins are fraternal twins.

In an embodiment of the present disclosure, the genotyping apparatus may further comprise: an amplifying unit, connected to the nucleic acid extracting unit and library constructing unit respectively, and suitable for subjecting the nucleic acid sample of the fetuses to amplifying, to obtain an amplification product containing the polymorphic site, for constructing the sequencing library. Thus, it may further improve efficiency of determining whether twins are fraternal twins.

In an embodiment of the present disclosure, the polymorphism is selected from a group consisting of STR, VNTR, RFLP, STS, SSCP, CAPS and SNP. Thus, it may further improve efficiency of determining whether twins are fraternal twins.

In an embodiment of the present disclosure, the polymorphic site is an STR polymorphic site being at least one selected from a group consisting of vWA, TPOX, D3S1358, D16S539, D5S818, CSF1PO, D11S2368, D2S1338, D8S1179, D13S317, D21S1437, D16S3391, Penta E, D12S1064, D12S391, D6S1043 and D19S433.

In an embodiment of the present disclosure, the amplifying unit is equipped with the primers, for subjecting the nucleic acid sample of the fetuses to amplifying by PCR, and for vWA, oligonucleotides shown as SEQ ID NO: 1 and SEQ ID NO: 2 are used as PCR primers; for TPOX, oligonucleotides shown as SEQ ID NO: 3 and SEQ ID NO: 4 are used as PCR primers; for D3S1358, oligonucleotides shown as SEQ ID NO: 5 and SEQ ID NO: 6 are used as PCR primers; for D16S539, oligonucleotides shown as SEQ ID NO: 7 and SEQ ID NO: 8 are used as PCR primers; for D5S818, oligonucleotides shown as SEQ ID NO: 9 and SEQ ID NO: 10 are used as PCR primers; for CSF1PO, oligonucleotides shown as SEQ ID NO: 11 and SEQ ID NO: 12 are used as PCR primers; for D11S2368, oligonucleotides shown as SEQ ID NO: 13 and SEQ ID NO: 14 are used as PCR primers; for D2S1338, oligonucleotides shown as SEQ ID NO: 15 and SEQ ID NO: 16 are used as PCR primers; for D8S1179, oligonucleotides shown as SEQ ID NO: 17 and SEQ ID NO: 18 are used as PCR primers; for D13S317, oligonucleotides shown as SEQ ID NO: 19 and SEQ ID NO: 20 are used as PCR primers; for D21S1437, oligonucleotides shown as SEQ ID NO: 21 and SEQ ID NO: 22 are used as PCR primers; for D16S3391, oligonucleotides shown as SEQ ID NO: 23 and SEQ ID NO: 24 are used as PCR primers; for Penta E, oligonucleotides shown as SEQ ID NO: 25 and SEQ ID NO: 26 are used as PCR primers; for D12S1064, oligonucleotides shown as SEQ ID NO: 27 and SEQ ID NO: 28 are used as PCR primers; for D12S391, oligonucleotides shown as SEQ ID NO: 29 and SEQ ID NO: 30 are used as PCR primers; for D6S1043, oligonucleotides shown as SEQ ID NO: 31 and SEQ ID NO: 32 are used as PCR primers; for D19S433, oligonucleotides shown as SEQ ID NO: 33 and SEQ ID NO: 34 are used as PCR primers. Thus, it may further improve efficiency of determining whether twins are fraternal twins.

Additional aspects and advantages of embodiments of present disclosure will be given in part in the following descriptions, become apparent in part from the following descriptions, or be learned from the practice of the embodiments of the present disclosure.

### BRIEF DESCRIPTION OF THE DRAWINGS

These and other aspects and advantages of embodiments of the present disclosure will become apparent and more readily appreciated from the following descriptions made with reference the accompanying drawings, in which:
Fig.1 is a flow chart showing a method of determining whether twins are fraternal twins according to an embodiment of the present disclosure;
Fig.2 is a flow chart showing a method of determining whether twins are fraternal twins according to another embodiment of the present disclosure;
Fig.3 is a schematic diagram showing a system for determining whether twins are fraternal twins according to an embodiment of the present disclosure;
Fig.4 is a schematic diagram showing a system for determining whether twins are fraternal twins according to another embodiment of the present disclosure;
Fig.5 is a genotyping result by gel electrophoresis according to an embodiment of the present disclosure; and
Fig.6 is a genotyping result by gel electrophoresis according to an embodiment of the present disclosure.

### DETAILED DESCRIPTION

Reference will be made in detail to embodiments of the present disclosure, the same or similar elements and the elements having same or similar functions are denoted by like reference numerals throughout the descriptions. The embodiments described herein with reference to drawings are explanatory, illustrative, and used to generally understand the present disclosure. The embodiments shall not be construed to limit the present disclosure.

It should note that terms such as "first" and "second" are used herein for purposes of description and are not intended to indicate or imply relative importance or significance. Thus, features defined with "first", "second" may explicitly or implicitly include one or more the features. Furthermore, in the description of the present disclosure, unless otherwise stated, "a/the plurality of" means two or more.

### Method of determining whether twins are fraternal twins

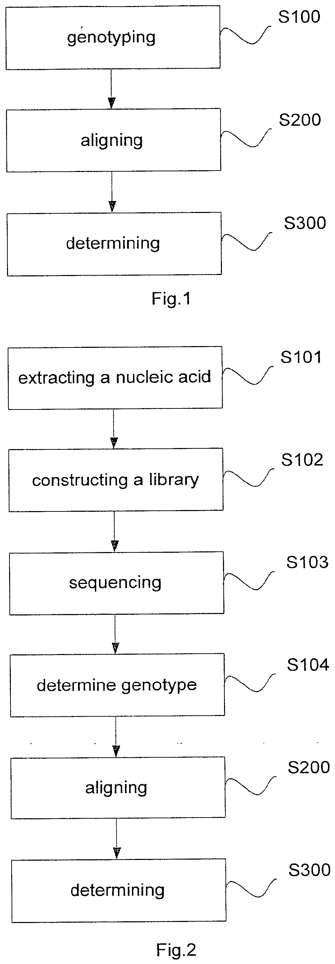

Embodiments of a first broad aspect of the present disclosure provide a method of determining whether twins are fraternal twins. According to embodiments of the present disclosure, referring to Fig.1, the method may comprise following steps:
S100 genotyping step: genotyping at least one polymorphic site of fetuses of the twins, to obtain polymorphism type of the fetuses.

According to embodiments of the present disclosure, methods of obtaining the polymorphism type of the fetuses are not subjecting to any special restrictions. According to some embodiments of the present disclosure, it may isolate a pregnant sample from a pregnant woman, and a nucleic acid sample of the fetuses may be further extracted from a pregnant sample. It should note that expression of "nucleic acid sample of fetuses", should be broadly understood, which may be any nucleic acid sample containing targeting polymorphism sites of fetus. On one hand, the nucleic acid sample of fetuses may be a whole genome nucleic acid sample of fetus, or may be a nucleic acid sample containing a nucleic acid fragment of fetus; on the other hand, the nucleic acid sample of fetuses may fully consist of fetal genetic materials, or may be a mixture of fetal genetic materials and pregnant genetic materials.

According to embodiments of the present disclosure, referring to Fig.2, the step of obtaining polymorphism type of the fetuses may further comprise:
Step 101: extracting a nucleic acid sample of the fetuses from a pregnant sample, which may effectively obtain the polymorphism type of the fetuses by sampling from a pregnant woman, and further effectively determine whether the twins are fraternal twins. According to embodiments of the present disclosure, types of the pregnant sample from which the nucleic acid sample is extracted are not subjected to any special restrictions. According to some embodiments of the present disclosure, the pregnant sample is selected from a group consisting of peripheral blood, pregnant woman urine and breast milk, preferably, the pregnant sample is peripheral blood. Thus, it may obtain the polymorphism type of the fetuses by noninvasive sampling from the pregnant woman, by which whether the twins are fraternal twins may be effectively determined, without causing adverse effect to fetal growth. According to embodiments of the present disclosure, methods and devices for extracting the nucleic acid sample of the fetuses from the pregnant sample are not subjected to any special restrictions, which may use commercial kit for extracting nucleic acid.
Step 102: after step 101, constructing a sequencing library for the nucleic acid sample of the fetuses. As for methods and processes of constructing a sequencing library for the nucleic acid sample, a person skilled in the art may appropriately select depending on different sequencing technology. Detailed process may refer to procedure provided by sequencer manufacturer, such as Illumina Company, for example, refer to Multiplexing Sample Preparation Guide (Part#1005361; Feb 2010) or Paired-End SamplePrep Guide (Part#1005063; Feb 2010) from Illumina Company, which are incorporated herein for reference. In an embodiment of the present disclosure, prior to the step of constructing the sequencing library, the method may further comprise: subjecting the nucleic acid sample of the fetuses to amplifying, to obtain an amplification product containing the polymorphic site, for constructing the sequencing library. Preferably, the amplification product has a length of 150 bp or less. Thus, it may further improve efficiency of determining whether twins are fraternal twins.

In an embodiment of the present disclosure, the polymorphism is selected from a group consisting of STR, VNTR, RFLP, STS, SSCP, CAPS and SNP. In an embodiment of the present disclosure, the polymorphic site is an STR polymorphic site being at least one selected from a group consisting of vWA, TPOX, D3S1358, D16S539, D5S818, CSF1PO, D11S2368, D2S1338, D8S1179, D13S317, D21S1437, D16S3391, Penta E, D12S1064, D12S391, D6S1043 and D19S433. Thus, it may further improve efficiency of determining whether twins are fraternal twins.

In an embodiment of the present disclosure, the nucleic acid sample of the fetuses is subjected to amplifying by PCR, and for vWA, oligonucleotides shown as SEQ ID NO: 1 and SEQ ID NO: 2 are used as PCR primers; for TPOX, oligonucleotides shown as SEQ ID NO: 3 and SEQ ID NO: 4 are used as PCR primers; for D3S1358, oligonucleotides shown as SEQ ID NO: 5 and SEQ ID NO: 6 are used as PCR primers; for D16S539, oligonucleotides shown as SEQ ID NO: 7 and SEQ ID NO: 8 are used as PCR primers; for D5S818, oligonucleotides shown as SEQ ID NO: 9 and SEQ ID NO: 10 are used as PCR primers; for CSF1PO, oligonucleotides shown as SEQ ID NO: 11 and SEQ ID NO: 12 are used as PCR primers; for D11S2368, oligonucleotides shown as SEQ ID NO: 13 and SEQ ID NO: 14 are used as PCR primers; for D2S1338, oligonucleotides shown as SEQ ID NO: 15 and SEQ ID NO: 16 are used as PCR primers; for D8S1179, oligonucleotides shown as SEQ ID NO: 17 and SEQ ID NO: 18 are used as PCR primers; for D13S317, oligonucleotides shown as SEQ ID NO: 19 and SEQ ID NO: 20 are used as PCR primers; for D21S1437, oligonucleotides shown as SEQ ID NO: 21 and SEQ ID NO: 22 are used as PCR primers; for D16S3391, oligonucleotides shown as SEQ ID NO: 23 and SEQ ID NO: 24 are used as PCR primers; for Penta E, oligonucleotides shown as SEQ ID NO: 25 and SEQ ID NO: 26 are used as PCR primers; for D12S1064, oligonucleotides shown as SEQ ID NO: 27 and SEQ ID NO: 28 are used as PCR primers; for D12S391, oligonucleotides shown as SEQ ID NO: 29 and SEQ ID NO: 30 are used as PCR primers; for D6S1043, oligonucleotides shown as SEQ ID NO: 31 and SEQ ID NO: 32 are used as PCR primers; for D19S433, oligonucleotides shown as SEQ ID NO: 33 and SEQ ID NO: 34 are used as PCR primers. Thus, it may further improve efficiency of determining whether twins are fraternal twins. The used PCR primers are shown in the Table 1 below:

**Table 1**

| Primer name* | Primer sequence | SEQ ID NO: |
|---|---|---|
| vWA-F | AATAATCAGTATGTGACTTGGATTGA | 1 |
| vWA-R | ATAGGATGGATGGATAGATGGA | 2 |
| TPOX-F | CTTAGGGAACCCTCACTGAATG | 3 |
| TPOX-R | GTCCTTGTCAGCGTTTATTTGC | 4 |
| D3S1.358-F | CAGAGCAAGACCCTGTCTCAT | 5 |
| D3S1358-R | TCAACAGAGGCTTGCATGTAT | 6 |
| D16S539-F | ATACAGACAGACAGACAGGTG | 7 |
| D16S539-R | GCATGTATCTATCATCCATCTCT | 8 |
| D5S818-F | GGGTGATTTTCCTCTTTGGT | 9 |
| D5S818-R | AACATTTGTATCTTTATCTGTATCCTTATTTAT | 10 |
| CSF1PO-F | ACAGTAACTGCCTTCATAGATAG | 11 |
| CSF1PO-R | GTGTCAGACCCTGTTCTAAGTA | 12 |
| D11S2368-F | ACAATGAGGTGCAAGAATGT | 13 |
| D11S2368-R | GTTAGATGGGTGGATGGATA | 14 |
| D2S1338-F | TGGAAACAGAAATGGCTTGG | 15 |
| D2S1338-R | GATTGCAGGAGGGAAGGAAG | 16 |
| D8S1179-F | TTTGTATTTCATGTGTACATTCGTATC | 17 |
| D8S1179-R | ACCTATCCTGTAGATTATTTTCACTGTG | 18 |
| D13S317-F | TCTGACCCATCTAACGCCTA | 19 |
| D13S317-R | CAGACAGAAAGATAGATAGATGATTGA | 20 |
| D21S1437-F | ATGTACATGTGTCTGGGAAGG | 21 |
| D21S1437-R | TACTGCCAACACTTGTCC | 22 |
| D16S3391-F | TCGCTCGCTCTATCTATCTATC | 23 |
| D16S3391-R | ACAGAACCAATAAGATGAG | 24 |
| Penta E-F | AGACTCAGTCTCAAAGAAA | 25 |
| Penta E-R | ATTGAGAAAACTCCTTAC | 26 |
| D12S1064-F | ACTACTCCAAGGTTCCAGCC | 27 |
| D12S1064-R | TGTGGTAGATAGATGATA | 28 |
| D12S391-F | GGATGCATAGGTAGATAGATAGA | 29 |
| D12S391-R | TAATAAATCCCCTCTCATCT | 30 |
| D6S1043-F | CAAGGATGGGTGGATCAATA | 31 |
| D6S1043-R | TTGTATGAGCCACTTCCCAT | 32 |
| D19S433-F | GCCTGGGCAACAGAATAAGA | 33 |
| D19S433-R | ATCTTCTCTCTTTCTTCCT | 34 |

| | | |
|---|---|---|
| *The primer name is used in an annotating pattern of site name + primer type. Taken D19S433-F and D6S1043-R as examples, D19S433-F represents a forward primer of D19S433, while D6S1043-R represents a reverse primer of D6S1043. | | |

Step 103, after the step 102, subjecting the sequencing library to sequencing, to obtain a sequencing result. After obtaining the sequencing library, the obtained sequencing library is applied to a sequencer for sequencing, to obtain a corresponding sequencing result, which consists of a plurality of sequencing data. According to embodiments of the present disclosure, methods and devices used for sequencing are not subjected to any special restrictions, including but not limited to Chain Termination Method; preferably a high-throughput sequencing method. Thus, utilizing characteristics of these sequencing apparatuses, the efficiency of determining whether twins are fraternal twins may be further improved, by which may improve accuracy and precision of subsequent analyzing with the sequencing data, particularly analyzing by statistical tests. Methods for high-throughput sequencing includes but not limited to a Next-Generation sequencing technology or a single molecule sequencing technology. The Next-Generation sequencing platform (Metzker ML. Sequencing technologies-the next generation. Nat Rev Genet. 2010 Jan; 11(1):31-46) includes but not limited to Illumina-Solexa (GATM, HiSeq2000TM, etc), ABI-Solid and Roche-454 (pyrosequencing) sequencing platforms; the single molecule sequencing platform (technology) includes but not limited to True Single Molecule DNA sequencing of Helicos Company, single molecule real-time (SMRT™) of Pacific Biosciences Company, and nanopore sequencing technology of Oxford Nanopore Technologies Company (Rusk, Nicole (2009-04-01). Cheap Third-Generation Sequencing. Nature Methods 6 (4): 244-245), etc. With gradual development of sequencing technology, a person skilled in the art may understand other sequencing methods and apparatuses may also be used for whole genome sequencing. According to specific examples of the present disclosure, the whole genome sequencing library may be subjected to sequencing by at least one selected from Illumina-Solexa, ABI-SOLiD, Roche-454 and a single molecule sequencing apparatus.

Step 104: after the step 103, determining the polymorphism type of the fetuses based on the sequencing result.

The sequencing result may be subjected to analyzing by conventional information analysis methods, to determine genotype of polymorphic site of fetuses. For example, the sequencing result may be subjected to aligning to a reference sequence using alignment software, to determine polymorphism genotype of fetuses in the polymorphic site. For example, SOAP software may be used for alignment. According to embodiments of the present disclosure, used reference sequence may be a human genome sequence. According to embodiments of the present disclosure, the number of used sites may be selected as required; the genotyping method is flexible, which may be based on sequence length or based on base sequence. According to embodiments of the present disclosure, the method of determining whether fetuses are monozygotic directly based on genetic information, by which determined result is accurate and reliable. According to embodiments of the present disclosure, the genotyping method may also performed by electrophoresis detection based on fragment length after PCR and sequence capture. Determination with the result is not only decided by specific genotyping result, but also decided by a proportion of each genotype among the total genotypes.

After the genotypes of polymorphic site in the fetuses are determined, obtained genotypes of polymorphic site in the fetuses may be aligned to corresponding genotype of polymorphic site in the parents thereof (Step 200, alignment step). For example, the whole genome samples of the parents may be subjected to sequencing parallel or in advance, to determine the genotypes of polymorphic site in the parents thereof for subsequent analysis. Then, whether the twins are fraternal twins is determined based on aligning result (Step 300, determination step). After the genotypes of polymorphic site in the fetuses are determined, if parental genotypes in a certain site are heterozygous and different with maternal site, at same time in the pregnant sample, for example both two of parental genotypes can be detected in the plasma, then the twins may be determined as fraternal twins. For example, taken STR as an example, genomic DNA of parents and pregnant plasma DNA are respectively subjected to genotyping of polymorphic site, the paternal genetic STR genotype, which is determined in view of pregnant STR genotype, is found in the plasma, if the paternal genetic STR genotype in a certain site is heterozygous and totally different with that of the pregnant STR genotype, and both two of the paternal genotypes can be detected in the plasma, then the twins may be determined as fraternal twins.

### System for determining whether twins are fraternal twins

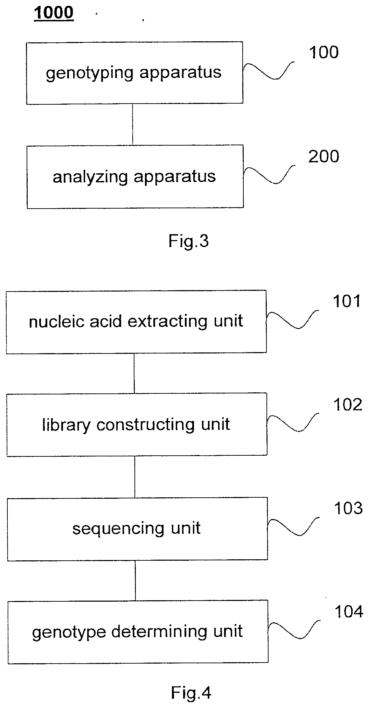

Embodiments of a second broad aspect of the present disclosure provide a system 1000 for determining whether twins are fraternal twins. Referring to Fig.3, according to embodiments of the present disclosure, the system may comprise: a genotyping apparatus 100 and an analyzing apparatus 200. According to embodiments of the present disclosure, the genotyping apparatus is used for subjecting at least one polymorphic site of fetuses of the twins to genotyping, to obtain polymorphism type of the fetuses. According to embodiments of the present disclosure, the analyzing apparatus is connected with the genotyping apparatus, and suitable for comparing the polymorphism type of the fetuses with corresponding polymorphic site of parents thereof, and determining whether the twins are the fraternal twins based on a comparing result. Terms concerning attachments, coupling and the like, such as "connected" and "interconnected", refer to a relationship wherein structures are secured or attached to one another either directly or indirectly through intervening structures, as well as both movable or rigid attachments or relationships, unless expressly described otherwise. Terms of "connected" should be broadly understood, which may refer to a direct connection or indirect connection, as long as achieving the above functional connection. Using the system may effectively implement the above-mentioned method of determining whether twins are fraternal twins. Thus, the system may effectively determine whether twins are fraternal twins. Taken STR as an example, a paternal genetic STR genotype is determined in a plasma in view of pregnant STR genotype, if the paternal genetic STR genotype in a certain site is heterozygous and totally different with that of the pregnant STR genotype, and both two of the paternal genotypes can be detected in the plasma, then the twins may be determined as fraternal twins.

Referring to Fig.4, in an embodiment of the present disclosure, the genotyping apparatus 100 may further comprise: a nucleic acid extracting unit 101, a library constructing unit 102, a sequencing unit 103 and a genotype determining unit 104. According to embodiments of the present disclosure, the nucleic acid extracting unit 101 is used for extracting a nucleic acid sample of the fetuses from a pregnant sample. The library constructing unit 102 connects to the library constructing unit 101, and is suitable for constructing a sequencing library for the nucleic acid sample of the fetuses. The sequencing unit 103 connects to the library constructing unit 102, and is suitable for subjecting the sequencing library sequencing, to obtain a sequencing result. And the genotype determining unit 104 connects to the sequencing unit, and is suitable for determining the polymorphism type of the fetuses.

As for methods and processes of constructing a sequencing library for the nucleic acid sample, a person skilled in the art may appropriately select depending on different sequencing technology. Detailed process may refer to procedure provided by sequencer manufacturer, such as Illumina Company, for example, refer to Multiplexing Sample Preparation Guide (Part#1005361; Feb 2010) or Paired-End SamplePrep Guide (Part#1005063; Feb 2010) from Illumina Company.

In an embodiment of the present disclosure, the genotyping apparatus further comprises an amplifying unit (not shown in Figs). The amplifying unit respectively connects to the nucleic acid extracting unit and the library constructing unit, and is suitable for subjecting the nucleic acid sample of the fetuses to amplifying, to obtain an amplification product containing the polymorphic site, for constructing the sequencing library. Thus, it may further improve efficiency of determining whether twin are fraternal twins. In an embodiment of the present disclosure, prior to the step of constructing the sequencing library, the method may further comprise: subjecting the nucleic acid sample of the fetuses to amplifying, to obtain an amplification product containing the polymorphic site, for constructing the sequencing library. Preferably, the amplification product has a length of 150 bp or less. Thus, it may further improve efficiency of determining whether twins are fraternal twins. In an embodiment of the present disclosure, the polymorphism is selected from a group consisting of STR, VNTR, RFLP, STS, SSCP, CAPS and SNP. Thus, it may further improve efficiency of determining whether twins are fraternal twins. In an embodiment of the present disclosure, the polymorphic site is an STR polymorphic site being at least one selected from a group consisting of vWA, TPOX, D3S1358, D16S539, D5S818, CSF1PO, D11S2368, D2S1338, D8S1179, D13S317, D21S1437, D16S3391, Penta E, D12S1064, D12S391, D6S1043 and D19S433. In an embodiment of the present disclosure, the amplifying unit is equipped with the primers, for subjecting the nucleic acid sample of the fetuses to amplifying by PCR, and for vWA, oligonucleotides shown as SEQ ID NO: 1 and SEQ ID NO: 2 are used as PCR primers; for TPOX, oligonucleotides shown as SEQ ID NO: 3 and SEQ ID NO: 4 are used as PCR primers; for D3S1358, oligonucleotides shown as SEQ ID NO: 5 and SEQ ID NO: 6 are used as PCR primers; for D16S539, oligonucleotides shown as SEQ ID NO: 7 and SEQ ID NO: 8 are used as PCR primers; for D5S818, oligonucleotides shown as SEQ ID NO: 9 and SEQ ID NO: 10 are used as PCR primers; for CSF1PO, oligonucleotides shown as SEQ ID NO: 11 and SEQ ID NO: 12 are used as PCR primers; for D11S2368, oligonucleotides shown as SEQ ID NO: 13 and SEQ ID NO: 14 are used as PCR primers; for D2S1338, oligonucleotides shown as SEQ ID NO: 15 and SEQ ID NO: 16 are used as PCR primers; for D8S1179, oligonucleotides shown as SEQ ID NO: 17 and SEQ ID NO: 18 are used as PCR primers; for D13S317, oligonucleotides shown as SEQ ID NO: 19 and SEQ ID NO: 20 are used as PCR primers; for D21S1437, oligonucleotides shown as SEQ ID NO: 21 and SEQ ID NO: 22 are used as PCR primers; for D16S3391, oligonucleotides shown as SEQ ID NO: 23 and SEQ ID NO: 24 are used as PCR primers; for Penta E, oligonucleotides shown as SEQ ID NO: 25 and SEQ ID NO: 26 are used as PCR primers; for D12S1064, oligonucleotides shown as SEQ ID NO: 27 and SEQ ID NO: 28 are used as PCR primers; for D12S391, oligonucleotides shown as SEQ ID NO: 29 and SEQ ID NO: 30 are used as PCR primers; for D6S1043, oligonucleotides shown as SEQ ID NO: 31 and SEQ ID NO: 32 are used as PCR primers; for D19S433, oligonucleotides shown as SEQ ID NO: 33 and SEQ ID NO: 34 are used as PCR primers. Thus, it may further improve efficiency of determining whether twins are fraternal twins.

Comparing with other existing methods of determining fraternal twins, the technical solutions according to embodiments of the present disclosure have following technical advantages, which embodies in noninvasive sampling, accuracy and genetic information amount obtainable:
1) the technical solutions according to embodiments of the present disclosure use noninvasive or minimally invasive sample methods as much as possible such as collecting pregnant peripheral blood or urine, etc, which may avoid inconvenience and abortion risk caused by conventional amniotic fluid or chorion sampling;
2) the technical solutions according to embodiments of the present disclosure determine fraternal twins based on a genotyping result one or more polymorphic sites, in which the site number may be selected as required; the genotyping method is flexible, which may be based on sequence length or based on base sequence, and the method of determining whether fetuses are monozygotic directly based on genetic information, by which determined result is accurate and reliable;
3) the technical solutions according to embodiments of the present disclosure may be more effectively used in diagnosing clinical diseases. Since the genetic materials brought by fraternal twins are not totally identical, each fetus of the twins should be determined separately when determining a certain disease; if the twins are determined as identical twins, there may be the possibility of misdiagnosis, by which may be used in better genetic develop-related research, better research on genetic mechanism of free fetal DNA in pregnant peripheral blood and existing status thereof, better guidance for noninvasive prenatal diagnosis.

Reference will be made in detail to examples of the present disclosure. It would be appreciated by those skilled in the art that the following examples are explanatory, and cannot be construed to limit the scope of the present disclosure. If the specific technology or conditions are not specified in the examples, a step will be performed in accordance with the techniques or conditions described in the literature in the art (for example, referring to J. Sambrook, et al. (translated by Huang PT), Molecular Cloning: A Laboratory Manual, 3rd Ed., Science Press) or in accordance with the product instructions. If the manufacturers of reagents or instruments are not specified, the reagents or instruments may be commercially available, for example, from Illumina company.

### EXAMPLE:

### General methods:

The method of determining whether twins are fraternal twins of the present disclosure includes:
1. noninvasive sampling a pregnant sample containing fetal genetic materials, extracting the fetal genetic materials;
2. extracting and purifying genomic DNA of fetuses' parents;
3. subjecting parents' genotypes and a mixture sampling from a pregnant woman and a fetuses thereof to polymorphic site genotyping;
4. aligning a new appearing polymorphic site genotype in the mixture sampling from a pregnant woman and a fetuses thereof to a genotyping result of paternal genome, to determine the genotype of the fetuses. By aligning the types of fetal genotype, if fetuses bring different paternal genotypes, then the fetuses are fraternal twins.

### Example 1

One plasma sample of a pregnant woman with twins was collected, while genomic DNA respectively from the couples were collected. The twins, implanted test-tube baby, had been clinical diagnosed as fraternal twins. 17 pairs of STR sites (vWA, TPOX, D3S1358, D16S539, D5S818, CSF1PO, D11S2368, D2S1338, D8S1179, D13S317, D21S1437, D16S3391, Penta E, D12S1064, D12S391, D6S1043, D19S433) commonly-used in paternity test were selected. PCR primers were designed which were shown in Table 1. The amplification product was required to have a length of 150 bp or less. Firstly, the genomic DNA of the parents were subjected to STR genotyping by means of taking the genomic DNA of the parents as a template, and subjecting the 14 pairs of primers to PCR amplification respectively using Phusion® high-fidelity DNA polymerase with an amplification system below:

| | |
|---|---|
| 2×Phusion PCR Master Mix | 25 µL |
| Forward primer | 5 µL |
| Reverse primer | 5 µL |
| DNA template | 15 µL |
| Total Volume | 50 µL |

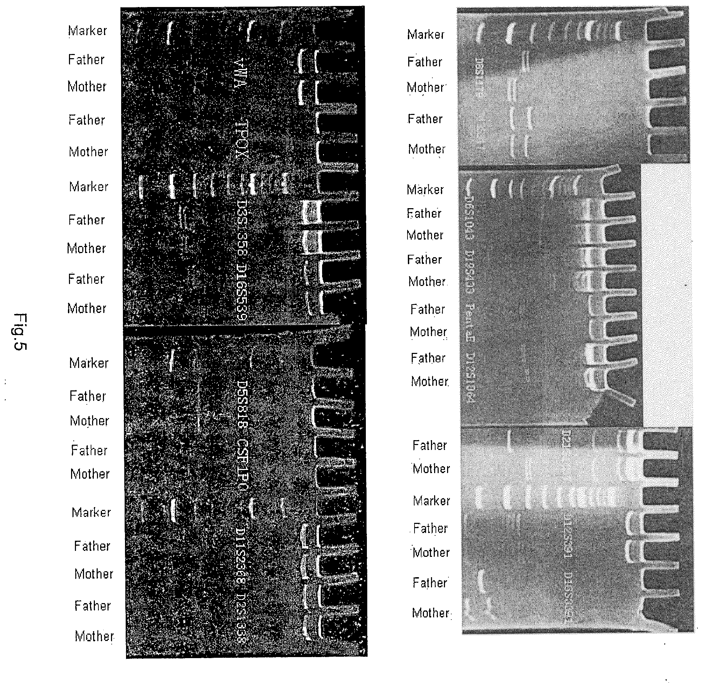

The PCR products were subjected to 12%PAGE electrophoresis genotyping, with 18 Voltage for 2 hours. As shown in Fig.5, the genotyping result illustrated that: except three sites (D6S1043, D19S433 and D12S391) showed a relative sever non-specific amplification, genotyping results of other sites were successful. There were 4 pairs of sites (D8S1179, vWA, D16S539 and D11S2368) showed that the parental genotypes were heterozygous and different with two copies of maternal genotype.

TIANamp Micro DNA Kit (TIANGEN) was used in extracting the plasma sample DNA for subsequent conventional library construction, with a detailed procedure:

### End-reparing:

| | |
|---|---|
| 10× T4 Polynucleotide kinase buffer | 10 µL |
| dNTPs (10 mM) | 2 µL |
| T4 DNA polymerase | 1 µL |
| Klenow fragments | 0.1 µL |
| T4 Polynucleotide kinase | 1 µL |
| DNA | 85 µL |
| ddH₂O | up to 100 µL |

After reacting at 20°C for 30 min, PCR Purification Kit (QIAGEN) was used in recycling end-repaired products. Then the recycled end-repaired products were finally dissolved in 34µL of EB buffer.

A reacting system for adding base A at end:

| | |
|---|---|
| 10× Klenow buffer | 5 µL |
| dATP (1 mM) | 10 µL |
| Klenow (3'-5' exo⁻) | 1 µL |
| DNA | 34 µL |

After incubating at 37°C for 30 min, obtained products were purified by MinElute® PCR Purification Kit (QIAGEN) and dissolved in 45 µL of EB buffer, to obtain DNA samples added with base A at end.

### Ligating adaptor:

| | |
|---|---|
| 2x Rapid DNA ligating buffer | 50 µL |
| PEI Adapter oligo-mix (20 µM) | 1 µL |
| T4 DNA ligase | 4 µL |
| DNA sample added with base A at end | 45 µL |

After reacting at 20°C for 15 min, PCR Purification Kit (QIAGEN) was used in recycling ligated products. The ligated products were finally dissolved in 15 µL of EB buffer.

### PCR reacting system:

| | |
|---|---|
| Ligated product | 15 µL |
| Phusion DNA Polymerase Mix | 25 µL |
| PCR primer (10 pmol/µL) | 2.5 µL |
| Index N (10 pmol/µL) | 2.5 µL |
| UltraPureTM Water | 5 µL |

Reacting procedure was shown as below:

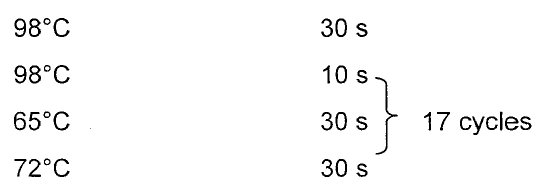

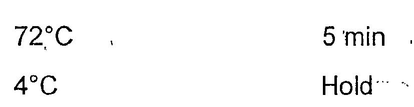

PCR Purification Kit (QIAGEN) was used in recycling PCR products, which were finally dissolved in 20 µL of EB buffer.

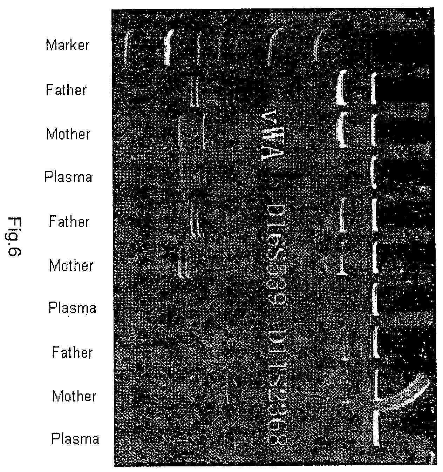

Taking the constructed plasma library as a template, 4 types of candidate STR sites were subjected to genotyping, the used genotyping method was consistent with the above. The result thereof was shown in Fig.6, vWA site, which was successfully amplified in the plasma sample, showed that the parental genomes were heterozygous and different with two copies of maternal genotype, it could be seen from the plasma genotyping result of parental two alleles, then two fetuses of the twin were regarded as respectively inheriting two different alleles of their biological father, which were determined as the fraternal twins

### Industrial Applicability

The technique solutions according to embodiments of the present disclosure may effectively determine whether twins are fraternal twins.

Reference throughout this specification to "an embodiment," "some embodiments," "one embodiment", "another example," "an example," "a specific examples," or "some examples," means that a particular feature, structure, material, or characteristic described in connection with the embodiment or example is included in at least one embodiment or example of the present disclosure. Thus, the appearances of the phrases such as "in some embodiments," "in one embodiment", "in an embodiment", "in another example, "in an example," "in a specific examples," or "in some examples," in various places throughout this specification are not necessarily referring to the same embodiment or example of the present disclosure. Furthermore, the particular features, structures, materials, or characteristics may be combined in any suitable manner in one or more embodiments or examples.

## Claims

1. A method of determining whether twins are fraternal twins, comprising the following steps:
genotyping at least one polymorphic site of fetuses of the twins, to obtain polymorphism type of the fetuses; wherein said step of genotyping comprises:
subjecting a nucleic acid sample of the fetuses extracted from maternal plasma to amplification, to obtain an amplified product containing an STR polymorphic site, for constructing a sequencing library,
wherein the STR polymorphic site is selected from a group consisting of vWA, TPOX, D3S1358, D16S539, D5S818, CSF1PO, D11S2368, D2S1338, D8S1179, D13S317, D21S1437, D16S3391, Penta E, D12S1064, D12S391, D6S1043 and D19S433; and
wherein the nucleic acid sample is amplified by PCR with primers as shown below, respectively:
for vWA, oligonucleotides shown as SEQ ID NO: 1 and SEQ ID NO: 2 are used as PCR primers;
for TPOX, oligonucleotides shown as SEQ ID NO: 3 and SEQ ID NO: 4 are used as PCR primers;
for D3S1358, oligonucleotides shown as SEQ ID NO: 5 and SEQ ID NO: 6 are used as PCR primers;
for D16S539, oligonucleotides shown as SEQ ID NO: 7 and SEQ ID NO: 8 are used as PCR primers;
for D5S818, oligonucleotides shown as SEQ ID NO: 9 and SEQ ID NO: 10 are used as PCR primers;
for CSF1PO, oligonucleotides shown as SEQ ID NO: 11 and SEQ ID NO: 12 are used as PCR primers;
for D11S2368, oligonucleotides shown as SEQ ID NO: 13 and SEQ ID NO: 14 are used as PCR primers;
for D2S1338, oligonucleotides shown as SEQ ID NO: 15 and SEQ ID NO: 16 are used as PCR primers;
for D8S1179, oligonucleotides shown as SEQ ID NO: 17 and SEQ ID NO: 18 are used as PCR primers;
for D13S317, oligonucleotides shown as SEQ ID NO: 19 and SEQ ID NO: 20 are used as PCR primers;
for D21S1437, oligonucleotides shown as SEQ ID NO: 21 and SEQ ID NO: 22 are used as PCR primers;
for D16S3391, oligonucleotides shown as SEQ ID NO: 23 and SEQ ID NO: 24 are used as PCR primers;
for Penta E, oligonucleotides shown as SEQ ID NO: 25 and SEQ ID NO: 26 are used as PCR primers;
for D12S1064, oligonucleotides shown as SEQ ID NO: 27 and SEQ ID NO: 28 are used as PCR primers;
for D12S391, oligonucleotides shown as SEQ ID NO: 29 and SEQ ID NO: 30 are used as PCR primers;
for D6S1043, oligonucleotides shown as SEQ ID NO: 31 and SEQ ID NO: 32 are used as PCR primers; and
for D19S433, oligonucleotides shown as SEQ ID NO: 33 and SEQ ID NO: 34 are used as PCR primers,
subjecting the sequencing library to sequencing, to obtain a sequencing result; determining a polymorphism type of the twin fetuses based on the sequencing result for the STR polymorphic site consisting of vWA, TPOX, D3S1358, D16S539, D5S818, CSF1PO, D11S2368, D2S1338, D8S1179, D13S317, D21S1437, D16S3391, Penta E, D12S1064, D12S391, D6S1043 and D19S433;
comparing the polymorphism type of the twin fetuses with corresponding polymorphic site of parents thereof; and
determining whether the twins are the fraternal twins based on a comparing result wherein it is determined that the twins are fraternal if the paternal genetic STR genotype in at least one site is heterozygous and is different with that of the maternal STR genotype, and both two of the paternal genotypes can be detected in the plasma.

2. The method of claim 1, wherein the amplification product has a length of 150 bp or less.

## Patentansprüche

1. Verfahren zum Bestimmen, ob Zwillinge zweieiige Zwillinge sind, umfassend die folgenden Schritte:
Genotypisieren von mindestens einer polymorphen Stelle der Föten von den Zwillingen, um einen Polymorphismustyp der Föten zu erhalten; wobei der Schritt des Genotypisierens das Folgende umfasst:
Unterziehen von einer Nukleinsäureprobe der Föten, die aus mütterlichem Plasma extrahiert ist, einer Amplifikation, um ein amplifiziertes Produkt zu erhalten, das eine polymorphe STR-Stelle enthält, um eine Sequenzierungsbibliothek zu konstruieren,
wobei die polymorphe SDR-Stelle aus der Gruppe ausgewählt ist, bestehend aus vWA, TPOX, D3S1358, D16S539, D5S818, CSF1PO, D11S2368, D2S1338, D8S1179, D13S317, D21S1437, D16S3391, Penta E, D12S1064, D12S391, D6S1043 und D19S433; und
wobei die Nukleinsäureprobe mittels PCR mit Primern, wie sie nachstehend gezeigt sind, amplifiziert wird, und zwar werden entsprechend:
für vWA Oligonukleotide, die als SEQ ID NO: 1 und SEQ ID NO: 2 gezeigt sind, als PCR-Primer verwendet;
für TPOX Oligonukleotide, die als SEQ ID NO: 3 und SEQ ID NO: 4 gezeigt sind, als PCR-Primer verwendet;
für D3S1358 Oligonukleotide, die als SEQ ID NO: 5 und SEQ ID NO: 6 gezeigt sind, als PCR-Primer verwendet;
für D16S539 Oligonukleotide, die als SEQ ID NO: 7 und SEQ ID NO: 8 gezeigt sind, als PCR-Primer verwendet;
für D5S818 Oligonukleotide, die als SEQ ID NO: 9 und SEQ ID NO: 10 gezeigt sind, als PCR-Primer verwendet;
für CSF1PO Oligonukleotide, die als SEQ ID NO: 11 und SEQ ID NO: 12 gezeigt sind, als PCR-Primer verwendet;
für D11S2368 Oligonukleotide, die als SEQ ID NO: 13 und SEQ ID NO: 14 gezeigt sind, als PCR-Primer verwendet;
für D2S1338 Oligonukleotide, die als SEQ ID NO: 15 und SEQ ID NO: 16 gezeigt sind, als PCR-Primer verwendet;
für D8S1179 Oligonukleotide, die als SEQ ID NO: 17 und SEQ ID NO: 18 gezeigt sind, als PCR-Primer verwendet;
für D13S317 Oligonukleotide, die als SEQ ID NO: 19 und SEQ ID NO: 20 gezeigt sind, als PCR-Primer verwendet;
für D21S1437 Oligonukleotide, die als SEQ ID NO: 21 und SEQ ID NO: 22 gezeigt sind, als PCR-Primer verwendet;
für D16S3391 Oligonukleotide, die als SEQ ID NO: 23 und SEQ ID NO: 24 gezeigt sind, als PCR-Primer verwendet;
für Penta E Oligonukleotide, die als SEQ ID NO: 25 und SEQ ID NO: 26 gezeigt sind, als PCR-Primer verwendet;
für D12S1064 Oligonukleotide, die als SEQ ID NO: 27 und SEQ ID NO: 28 gezeigt sind, als PCR-Primer verwendet;
für D12S391 Oligonukleotide, die als SEQ ID NO: 29 und SEQ ID NO: 30 gezeigt sind, als PCR-Primer verwendet;
für D6S1043 Oligonukleotide, die als SEQ ID NO: 31 und SEQ ID NO: 32 gezeigt sind, als PCR-Primer verwendet; und
für D19S433 Oligonukleotide, die als SEQ ID NO: 33 und SEQ ID NO: 34 gezeigt sind, als PCR-Primer verwendet,
Unterziehen von der Sequenzierungsbibliothek einer Sequenzierung, um ein Sequenzierungsergebnis zu erhalten;
Bestimmen von einem Polymorphismustyp der Zwillingsföten auf Basis von dem Sequenzierungsergebnis für die polymorphe STR-Stelle, bestehend aus vWA, TPOX, D3S1358, D16S539, D5S818, CSF1PO, D11S2368, D2S1338, D8S1179, D13S317, D21S1437, D16S3391, Penta E, D12S1064, D12S391, D6S1043 und D19S433;
Vergleichen von dem Polymorphismustyp der Zwillingsföten mit entsprechenden polymorphen Stellen von deren Eltern; und
Bestimmen auf Basis von einem Vergleichsergebnis, ob die Zwillinge zweieiige Zwillinge sind, wobei bestimmt wird, dass die Zwillinge zweieiige sind, wenn der väterliche genetische STR-Genotyp in mindestens einer Stelle heterozygot ist und sich von dem des mütterlichen STR-Genotyps unterscheidet und beide zwei des väterlichen Genotyp in dem Plasma detektiert werden können.

2. Verfahren nach Anspruch 1, wobei das Amplifikationsprodukt eine Länge von 150 bp oder weniger aufweist.

## Revendications

1. Procédé pour déterminer si des jumeaux sont des jumeaux fraternels, comprenant les étapes suivantes :
génotypage d'au moins un site polymorphe des foetus des jumeaux, pour obtenir le type de polymorphisme des foetus ; dans lequel ladite étape de génotypage comprend :
la soumission d'un échantillon d'acide nucléique des foetus extrait du plasma maternel en vue de l'amplification, pour obtenir un produit amplifié contenant un site polymorphe STR, pour construire une banque de séquençage,
dans lequel le site polymorphe STR est choisi dans un groupe constitué de vWA, TPOX, D3S1358, D16S539, D5S818, CSF1PO, D11S2368, D2S1338, D8S1179, D13S317, D21S1437, D16S3391, Penta E, D12S1064, D12S391, D6S1043 et D19S433 ; et
dans lequel l'échantillon d'acide nucléique est amplifié par amplification en chaîne par polymérase avec des amorces telles qu'illustrées ci-dessous, respectivement :
pour vWA, les oligonucléotides représentés par les SEQ ID N° : 1 et SEQ ID N° : 2 sont utilisés en tant qu'amorces d'amplification en chaîne par polymérase ;
pour TPOX, les oligonucléotides représentés par les SEQ ID N° : 3 et SEQ ID N° : 4 sont utilisés en tant qu'amorces d'amplification en chaîne par polymérase ;
pour D3S1358, les oligonucléotides représentés par les SEQ ID N° : 5 et SEQ ID N° : 6 sont utilisés en tant qu'amorces d'amplification en chaîne par polymérase ;
pour D16S539, les oligonucléotides représentés par les SEQ ID N° : 7 et SEQ ID N° : 8 sont utilisés en tant qu'amorces d'amplification en chaîne par polymérase ;
pour D5S818, les oligonucléotides représentés par les SEQ ID N° : 9 et SEQ ID N° : 10 sont utilisés en tant qu'amorces d'amplification en chaîne par polymérase ;
pour CSF1PO, les oligonucléotides représentés par les SEQ ID N° : 11 et SEQ ID N° : 12 sont utilisés en tant qu'amorces d'amplification en chaîne par polymérase ;
pour D11S2368, les oligonucléotides représentés par les SEQ ID N° : 13 et SEQ ID N° : 14 sont utilisés en tant qu'amorces d'amplification en chaîne par polymérase ;
pour D2S1338, les oligonucléotides représentés par les SEQ ID N° : 15 et SEQ ID N° : 16 sont utilisés en tant qu'amorces d'amplification en chaîne par polymérase ;
pour D8S1179, les oligonucléotides représentés par les SEQ ID N° : 17 et SEQ ID N° : 18 sont utilisés en tant qu'amorces d'amplification en chaîne par polymérase ;
pour D13S317, les oligonucléotides représentés par les SEQ ID N° : 19 et SEQ ID N° : 20 sont utilisés en tant qu'amorces d'amplification en chaîne par polymérase ;
pour D21S1437, les oligonucléotides représentés par les SEQ ID N° : 21 et SEQ ID N° : 22 sont utilisés en tant qu'amorces d'amplification en chaîne par polymérase ;
pour D16S3391, les oligonucléotides représentés par les SEQ ID N° : 23 et SEQ ID N° : 24 sont utilisés en tant qu'amorces d'amplification en chaîne par polymérase ;
pour Penta E, les oligonucléotides représentés par les SEQ ID N° : 25 et SEQ ID N° : 26 sont utilisés en tant qu'amorces d'amplification en chaîne par polymérase ;
pour D12S1064, les oligonucléotides représentés par les SEQ ID N° : 27 et SEQ ID N° : 28 sont utilisés en tant qu'amorces d'amplification en chaîne par polymérase ;
pour D12S391, les oligonucléotides représentés par les SEQ ID N° : 29 et SEQ ID N° : 30 sont utilisés en tant qu'amorces d'amplification en chaîne par polymérase ;
pour D6S1043, les oligonucléotides représentés par les SEQ ID N° : 31 et SEQ ID N° : 32 sont utilisés en tant qu'amorces d'amplification en chaîne par polymérase ; et
pour D19S433, les oligonucléotides représentés par les SEQ ID N° : 33 et SEQ ID N° : 34 sont utilisés en tant qu'amorces d'amplification en chaîne par polymérase,
la soumission de la banque de séquençage à un séquençage, pour obtenir un résultat de séquençage ;
la détermination d'un type de polymorphisme des foetus des jumeaux sur la base du résultat de séquençage pour le site polymorphe STR constitué de vWA, TPOX, D3S1358, D16S539, D5S818, CSF1PO, D11S2368, D2S1338, D8S1179, D13S317, D21S1437, D16S3391, Penta E, D12S1064, D12S391, D6S1043 et D19S433 ;
la comparaison du type de polymorphisme des foetus des jumeaux avec le site polymorphe correspondant des parents de ceux-ci ; et
le fait de déterminer si les jumeaux sont les jumeaux fraternels sur la base d'un résultat de comparaison
dans lequel il est déterminé que les jumeaux sont fraternels si le génotype STR génétique paternel dans au moins un site est hétérozygote et est différent de celui du génotype STR maternel, et les deux génotypes paternels peuvent être détectés dans le plasma.

2. Procédé selon la revendication 1, dans lequel le produit de l'amplification a une longueur de 150 pb ou moins.
